(19) Europäisches Patentamt
European Patent Office
Office européen des brevets

(11) Veröffentlichungsnummer: **0 295 327**
**A2**

# (12) EUROPÄISCHE PATENTANMELDUNG

(21) Anmeldenummer: **87110346.1**

(51) Int. Cl.⁴: **C07D 235/32**

(22) Anmeldetag: **17.07.87**

(30) Priorität: **13.06.87 DE 3719783**

(43) Veröffentlichungstag der Anmeldung:
**21.12.88 Patentblatt 88/51**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Lachhein, Stephen, Dr.**
**Kiedricher Strasse 18**
**D-6238 Hofheim am Taunus(DE)**
Erfinder: **Mildenberger, Hilmar, Dr.**
**Fasanenstrasse 24**
**D-6233 Kelkheim (Taunus)(DE)**
Erfinder: **Ressel, Hans-Joachim**
**Lorsbacher Strasse 17**
**D-6234 Hattersheim am Main(DE)**

## (54) Verfahren zur Herstellung von 5-Phenylsulfinyl-1H-1-(methoxycarbonylamino)-benzimidazol.

(57) Es wird ein hochselektives, billiges und quantitatives Verfahren zur Herstellung von 5-Phenylsulfinyl-1H-2-(methoxycarbonylamino)-benzimidazol (Oxfendazol) durch Umsetzung von 5-Phenylmercapto-1H-2-(methoxycarbonylamino)-benzimidazol mit Wasserstoffperoxid in Gegenwart eines oder mehrerer aliphatischer $C_1$-$C_6$-Alkohole unter Zufügen einer starken, nicht oxidierenden Mineralsäure beschrieben.

EP 0 295 327 A2

## Verfahren zur Herstellung von 5-Phenylsulfinyl-1H-2-(methoxycarbonylamino)-benzimidazol

5-Phenylsulfinyl-1H-2-(methoxycarbonylamino)-benzimidazol (Kurzbezeichnung: Oxfendazol) der Formel I ist ein wertvolles Anthelmintikum mit einer großen Wirkungsbreite, das sich vorzüglich zur Bekämpfung von parasitären Erkrankungen bei Mensch und Tier eignet (DE-PS 23 63 351).

Zur Herstellung von z.B. 5-Phenylsulfinyl-1H-2-(methoxycarbonylamino)-benzimidazol I

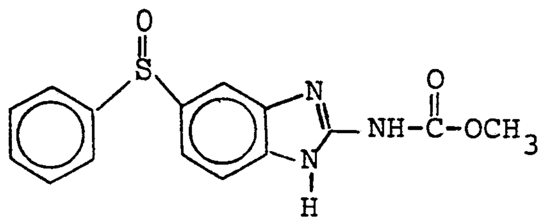

I

wird 5-Phenylmercapto-1H-2-(methoxycarbonylamino)-benzimidazol (Kurzbezeichnung: Fenbendazol) II

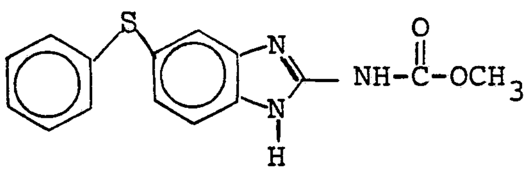

II

mit Wasserstoffperoxid in Essigsäure umgesetzt (DE-OS 23 34 631, DE-OS 24 32 631). Nachteil dieser Verfahrensweise ist das durch Überoxidation als Nebenprodukt sich bildende 5-Phenylsulfonyl-1H-2-(methoxycarbonyl-amino)-benzimidazol.

Bekannt ist, daß Thioether generell mit Wasserstoffperoxid in organischen Säuren wie z.B. Ameisensäure zum Sulfoxid oxidiert werden können (K. Undheim, Act.Chem.Scand., 24, (1970), 3429).

Ferner ist die Oxidation von Thioethern mit Wasserstoffperoxid in Methanol beschrieben (J. Drabowicz et al., Synth. Commun. 11(12), (1981), S. 1025 ff). Selektive Oxidationen (keine Sulfonbildung) und hohe Ausbeuten erhält man jedoch nur bei der Synthese von Dialkyl- und Aryl-Alkyl-Sulfoxiden. Für die Oxidation von Diarylthioethern ist dieses Verfahren nicht geeignet.

Außerdem gelingt die Oxidation mit oxidierenden Säuren wie Salpetersäure (Houben-Weyl, Bd. 4/1a (1981), S.736 ff) und $H_2O_2$/Schwefelsäure (EP-A 91052). Nachteile dieser Oxidationsverfahren sind die Bildung des Sulfons (Überoxidation→ niedrige Selektivität) als unerwünschtes Nebenprodukt und die häufig sehr schlechten Ausbeuten (G. Barbieri et al., J. Chem. Soc.[C], (1968), S. 659). Selektive Oxidationen in hohen Ausbeuten gelingen nur mit teuren Reagenzien wie z.B. Titantrichlorid (Y. Watanabe et al., Synthesis, (1981), S. 204) und Brom in Zweiphasensystem mit chlorierten Kohlenwasserstoffen (J. Drabowicz et al., Synthesis, (1979), S. 39).

Die vorliegende Erfindung betrifft ein hochselektives, billiges und einfaches Verfahren zur Herstellung von 5-Phenylsulfinyl-1H-2-(methoxycarbonylamino)-benzimidazol (I).

Es wurde nun für heterocyclische Thioether überraschenderweise gefunden, daß man einen hochselektiven Reaktionsablauf in quantitativer Ausbeute erhält, wenn man die Oxidationsreaktion mit Wasserstoffperoxid in Gegenwart aliphatischer Alkohole unter Einsatz einer nicht oxidierenden Mineralsäure durchführt.

Gegenstand der Erfindung ist daher ein Verfahren zur Herstellung von Oxfendazol durch Umsetzung von 5-Phenylmercapto-1H-2-(methoxycarbonylamino)-benzimidazol mit Wasserstoffperoxid, dadurch gekennzeichnet, daß man die Reaktion in Mineralsäure in Gegenwart eines oder mehrerer aliphatischer $C_1$-$C_6$-Alkohole durchführt.

Das erfindungsgemäße Verfahren zeichnet sich dadurch besonders aus, daß die gesamte Menge an Wasserstoffperoxid am Anfang der Reaktion in einer Portion zum Reaktionsgemisch zugegeben werden kann. Trotz dieses großen Überschusses an Wasserstoffperoxid wird unter diesen Bedingungen aus dem entstehenden Sulfoxid (Oxfendazol) kein 5-Phenylsulfonyl-1H-2-(methoxycarbonylamino)-benzimidazol gebildet.

Der besondere Vorteile dieses Verfahrens ergibt sich somit in einer völlig freien Wahl des Zugabemodus des Oxidationsmittels, was in Hinblick auf eine technische Durchführung von entscheidender Bedeutung ist.

Als aliphatische Alkohole sind z.B. $C_1$-$C_6$-Alkanole, besonders Methanol von Bedeutung.

Als Mineralsäuren kommen nicht oxidierende Säuren, wie z.B. Salzsäure, Bromwasserstoffsäure und Phosphorsäure in Frage.

Als Oxidationsmittel wird Wasserstoffperoxid verwendet, das äquimolar und bis zu einem doppelten äquimolaren Überschuß eingesetzt werden kann. Der pH-Bereich und der Temperaturbereich kann in weiten Grenzen variieren. Das Verfahren wird jedoch bevorzugt bei einem pH-Wert zwischen -1,0 bis 2,0 und einer Reaktionstemperatur von 0° bis 80° C durchgeführt.

Zur Durchführung der Reaktion wird zweckmäßigerweise 5Phenylmercapto-1H-2-(methoxycarbonylamino)-benzimidazol in dem Alkohol gelöst oder aufgeschlämmt und bei 0° C bis 80° C, vorzugsweise bei 20° C bis 60° C mit der nicht oxidierenden Mineralsäure versetzt, wobei ein pH-Wert von -1,0 bis 2,0, vorzugsweise von 0 bis 1,5 eingestellt wird. Die Zugabe kann auch in umgekehrter Reihenfolge geschehen. Danach wird mit der äquimolaren dis doppelt äquimolaren Menge Wasserstoffperoxid versetzt. Das Wasserstoffperoxid wird als 5 bis 50 %ige wässrige Lösung eingesetzt und kann zu Beginn der Reaktion in der gesamten Menge oder in beliebigen Portionen über den gesamten Versuchsablauf zugegeben werden.

Es ist ferner zweckmäßig, das Verfahren unter Inertgasatmosphäre, beispielsweise unter Stickstoff durchzuführen, um störende Einflüsse von Sauerstoff auf die Reaktion zu vermeiden.

Das erfindungsgemäße Verfahren zeichnet sich dadurch aus, daß die Oxidation von Thioethern zum Sulfoxid völlig selektiv (keine Sulfonbildung) und quantitativ abläuft, und die Bildung des Sulfoxides nicht vom Zugabemodus des Oxidationsmittels abhängig ist. Es werden keine organischen Säuren verwendet. Das Abwasser ist nicht fischtoxisch und kann leicht abgebaut werden. Das erhaltene Oxfendazol ist spezifikationsgerecht. Das erfindungsgemäße Verfahren wird nachfolgend an einigen Beispielen erläutert.

<u>Beispiel 1</u>

Zu einer Lösung von 186,4 g Fenbendazol in 750 ml Methanol, 250 g konz. Salzsäure und 630 ml Wasser werden bei 50° C und einem pH-Wert von 0,3-0,4 61,7 einer 35 %igen Wasserstoffperoxidlösung, verdünnt in 62 g Wasser, innerhalb von 2 Stunden unter Stickstoffatmosphäre zugetropft.

Das Reaktionsgemisch wird für 6 Stunden bei 50° C nachgerührt und mit 3,0 g Natriumsulfit versetzt. Nach Abkühlen auf Raumtemperatur wird mit 600 g Wasser verdünnt und das Reaktionsgemisch mit 520 g 20 %iger Natronlauge auf pH 6 eingestellt. Das ausgefallene Rohprodukt wird abfiltriert und mit Wasser gewaschen. Nach dem Trocknen im Trockenschrank verbleiben 194 g Oxfendazol mit einer Reinheit von 98,6 %. Die Ausbeute bezogen auf das eingesetzte Fenbendazol ist 98,5 % d. Th.

Das Produkt ist weiß und enthält keine färbende Bestandteile. Es wird kein Fenbendazol und kein 5-Phenylsulfonyl-1H-2-(methoxycarbonylamino)-benzimidazol im Produkt gefunden. Aus dem Waschwasser werden 750 ml Methanol nahezu quantitativ zurückgewonnen, was im nächsten Ansatz wiederum einsetzbar ist. Das Abwasser zeigt keine Fischtoxizität und ist biologisch leicht abbaubar.

<u>Beispiel 2</u>

Es werden 186,4 g Fenbendazol in 700 ml Methanol, 513 g 48 %iger Bromwasserstoffsäure und 400 ml Wasser bei 60° C gelöst und bei einem pH-Wert von -0,5 mit 65,4 g einer 35 %igen Wasserstoffperoxidlösung versetzt. Die Reaktionslösung wird für 7 Stunden auf 60° C erhitzt und danach mit 10,0 g Natriumsulfit versetzt. Nach dem Abkühlen auf Raumtemperatur wird das Reaktionsgemisch in 20 %ige Natronlauge eingebracht, wobei das Produkt auskristallisiert. Nach dem Trocknen im Vakuum verbleiben 194,5 g Oxfendazol, was einer Ausbeute von 98,8 % bezogen auf das eingesetzte Fenbendazol entspricht. Die Reinheit beträgt 98,7 %.

<u>Beispiel 3</u>

Unter Stickstoffatmosphäre werden 372,8 g Fenbendazol in 1300 ml Methanol gelöst und mit 500 g konz. Salzsäure versetzt. Bei 40° C werden zu dieser Lösung innerhalb von 5 Stunden 130 g einer 35 %igen Wasserstoffperoxidlösung getropft und für weitere 3 Stunden bei 40° C erhitzt. Nach Zugabe von 20

g Natriumsulfit wird mit 50 %iger Natronlauge auf pH 4 eingestellt und das ausgefallene Produkt abgesaugt. Nach dem Waschen mit Wasser und Trocknen im Vakuumtrockenschrank werden 390,0 g Oxfendazol erhalten. Die Reinheit beträgt 98,6 % und die Ausbeute 99,0 % d. Th. bezegen auf das eingesetzte Fenbendazol.

Der der Anmeldung zugrundeliegende Stand der Technik wird durch die folgenden Vergleichsbeispiele näher erläutert.

## Vergleichsbeispiel 1

Oxidation mit Wasserstoffperoxid in Methanol ohne Säurezusatz (gemäß J. Drabowicz et al., Synth. Commun. 11(2), (1981), S. 1025 ff).

372,8 g Fenbendazol werden in 300 ml Methanol gelöst und bei 50 °C mit 130 g einer 35 %igen Wasserstoffperoxidlösung innerhalb von 3 Stunden versetzt. Nach insgesamt 7 Stunden Reaktionszeit werden 10 g Natriumsulfit zugegeben und nach dem Abdestillieren von 1000 ml Methanol das Rohprodukt abfiltriert.

Nach dem Waschen und Trocknen im Vakuumtrockenschrank werden 381,0 g Produkt mit folgender Zusammensetzung abfiltriert:

43,4 % Fenbendazol
29,2 % Oxfendazol
27,3 % 5-Phenylsulfonyl-1H-2-(methoxycarbonylamino)-benzimidazol

Die Ausbeute beträgt 13,9 % d. Th. bezogen auf das eingesetzte Fenbendazol.

## Vergleichsbeispiel 2

Oxidation mit Wasserstoffperoxid in Essigsäure (gemäß DE-OS 23 34 631, DE-OS 24 32 631) 186,4 g Fenbendazol werden in 1600 ml Eisessig aufgeschlämmt und bei Raumtemperatur mit 593 ml einer 30 %igen Wasserstoffperoxid-Lösung versetzt. Nach 1 stündigem Rühren bei Raumtemperatur gibt man die Lösung in 3000 ml Wasser, filtriert das ausgefallene Produkt ab, wäscht mit Wasser und Methanol und trocknet anschließend im Vakuumtrockenschrank. Es verbleiben als Rohprodukt 132,7 g Oxfendazol mit einer Reinheit von 91,2 %, entsprechend einer Ausbeute von 67,4 %. Der Gehalt an 5-Phenylsulfonyl-1H-2-(methoxycarbonylamino)-benzimidazol beträgt 7,2 %.

Die Beispiele des erfindungsgemäßen Verfahrens zeigen, daß deutlich höhere Ausbeuten (98,5 - 99 %) und bessere Reinheiten (98,6 - 98,7 % d. Th.) der Produkte gegenüber den in den Vergleichsbeispielen beschriebenen Verfahren erzielt werden.

## Ansprüche

1. Verfahren zur Herstellung von 5-Phenylsulfinyl-1H-2(methoxycarbonylamino)-benzimidazol durch Umsetzung von 5-Phenylmercapto-1H-2-(methoxycarbonylamino)-benzimidazol mit Wasserstoffperoxid, dadurch gekennzeichnet, daß man die Reaktion in Mineralsäure in Gegenwart eines oder mehrerer aliphatischer $C_1$-$C_6$-Alkohole durchführt.

2. Verfahren gemäß Anspruch 1, dadurch gekennzeichnet, daß man als Mineralsäure Salzsäure, Bromwasserstoffsäure oder Phosphorsäure verwendet.

3. Verfahren gemäß einem oder mehrerer der Ansprüche 1 oder 2, dadurch gekennzeichnet, daß man als Lösungsmittel aliphatische $C_1$-$C_6$-Alkanole verwendet.

4. Verfahren gemäß einem oder mehrerer der Ansprüche 1 bis 3, dadurch gekennzeichnet, daß man Salzsäure und Methanol verwendet.

5. Verfahren gemäß einem oder mehrerer der Ansprüche 1 bis 4, dadurch gekennzeichnet, daß man die Umsetzung bei 0 ° bis 80 °C durchführt.

6. Verfahren gemäß einem oder mehrerer der Ansprüche 1 bis 5, dadurch gekennzeichnet, daß man die Umsetzung bei pH -1,0 bis 2,0 durchführt.

7. Verfahren gemäß einem oder mehrerer der Ansprüche 1 bis 6, dadurch gekennzeichnet, daß man die Umsetzung unter Inertgasatmosphäre durchführt.